# EUROPEAN PATENT APPLICATION

(11) **EP 2 390 245 A1**
(43) Date of publication of application: **30.11.2011**
(21) Application number: 10450093.9
(22) Date of filing: 26.05.2010
(51) Int. Cl.: C07C 211/40, C07C 247/24, C07C 263/12, C07C 265/10, C07C 269/08, C07C 271/24, C07D 303/36, C07C 61/22, C07C 327/16, C07C 247/22

(54) **Enantiomerically pure amines**

(71) Applicant: Nabriva Therapeutics AG, 1112 Wien (AT)
(72) Inventor: Mang, Rosemarie, 1220 Wien (AT); Heilmayer, Werner, 7034 Zillingtal (AT); Spence, Lee, 1050 Wien (AT); Bulusu, Atchyuta Rama Chandra Murty, 2380 Perchtoldsdorf (AT)
(74) Representative: Schwarz, Albin

(57) **Abstract**

A compound of formula 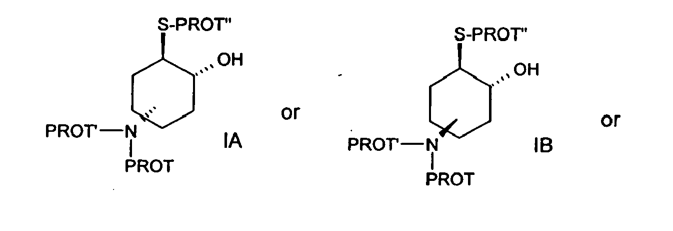 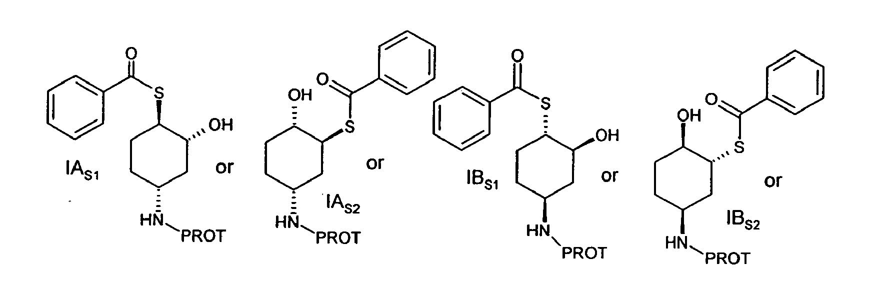 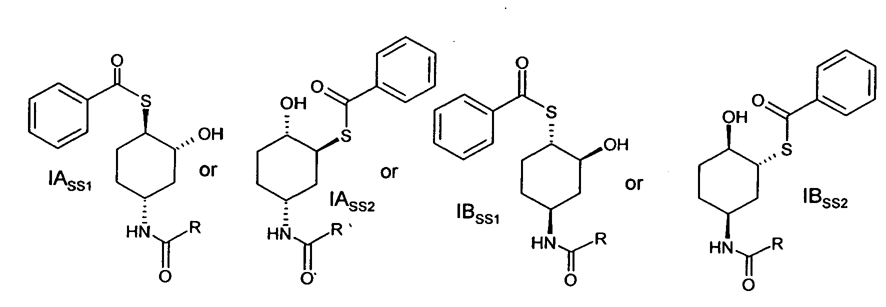 wherein PROT, PROT' and R have various meanings, processes for its production and production of intermediates in stereoisomerically pure form, and its use for the production of pharmaceutically active compounds.

## Description

The present invention relates to enantiomerically pure amines, such as cyclohexyl amines and production processes thereof.

Organic compounds, such as cyclohexyl amines containing an asymmetric carbon atom may exist in the form of enantiomers, diastereoisomers and mixtures thereof, e.g. racemates. Such compounds may exist in the (R)-, (S)- or (R,S)-configuration. For pharmaceutical use it is often vital to have an active compound comprising an asymmetric carbon atom in one of the enantiomerically pure forms, since one isomer may differ, e.g. in several aspects from another isomer, e.g. one isomer may be more active than the other isomer. Separation of isomers is often burdensome. Chromatography which, for example, may be useful for isomeric separation, is on technical scale not easy to carry out and often needs sophisticated and expensive means.

Surprisingly it was now found that enantiomerically pure cycloalkyl amines with interesting pharmaceutical characteristics may be produced on technical scale. In the course of the production process according to the present invention interesting intermediates may be isolated, e.g. in solid, such as in crystalline form.

Enantiomerically pure cyclohexyl amines which were found according to the present invention include enantiomerically pure cyclohexyl amines, which optionally are attached to a pleuromutilin, e.g. which are attached via a thiomethylcarbonyl group to a mutilin, e.g. which are attached via a thiomethylcarbonyl group to the oxygen in position 14 of the mutilin ring. Pleuromutilins comprising such enantiomerically pure cyclohexyl amines have been found to be pharmaceutically interesting, such as compounds as disclosed in W02008/113089 which are described to be useful for treating microbial diseases.
In one aspect the present invention provides a compound of formula 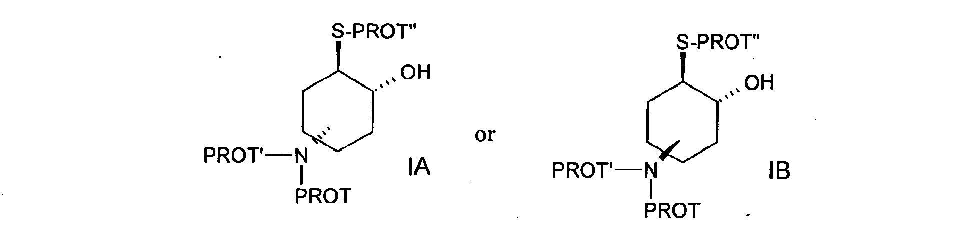 wherein PROT is an amine protecting group and PROT' is hydrogen; or PROT and PROT' together with the nitrogen atom to which they are attached form a heterocyclic ring as an amine protecting group,
e.g. wherein the -N(PROT)(PROT') group is either in position 3 or in position 4 in the cyclohexyl ring relative to the thio-group, preferably in position 4; and PROT" is a thiol protecting group, preferably (C₆₋₁₂)arylcarbonyl, such as benzoyl.

Amine protecting groups PROT and PROT' are known and may be obtained as appropiate, e.g. according to a method as conventional.

Preferably PROT is an amine protecting group and PROT' is hydrogen.

Well known PROT groups include e.g. groups connected to the amine via an oxy- or carbonyloxy-, carbonyl- or oxycarbonyl-, SO₂-, arylalkyl, e.g. including triphenylmethyl, or C=N double bond.

PROT' e.g. includes a group which is part of a heterocyclic ring comprising the nitrogen of the amine to be protected and PROT and PROT'; e.g. the amine where PROT and PROT' are attached is part of a phthalimide ring.

Groups "PROT" or "PROT"', respectively, are removable under acidic, basic, hydrogenating, oxidative or reductive conditions, e.g. by hydrogenolysis, treatment with an acid, a base, a hydride, a sulfide.

For example, PROT' is hydrogen, and PROT is, e.g. selected from
- benzyloxycarbonyl (Cbz), removable e.g. by hydrogenolysis,
- p-methoxybenzylcarbonyl (Moz or MeOZ), removable e.g. by hydrogenolysis,
- *tert*-butyloxycarbonyl (BOC), removable e.g. by treatment with a strong acid, such as HCl, H₃P0₄, or CF₃COOH,
- 9-fluorenylmethyloxycarbonyl (FMOC), removable e.g. by treatment with a base, such as piperidine,
- benzyl (Bn), removable e.g. by hydrogenolysis;
- p-methoxybenzyl (PMB), removable e.g. by hydrogenolysis;
- 3,4-dimethoxybenzyl (DMPM), removable e.g. by hydrogenolysis;
- p-methoxyphenyl (PMP), removable e.g. by treatment with ammonium cerium(IV) nitrate (CAN),
- tosyl (Tos), removable e.g. by treatment with concentrated acid, such as HBr, H₂S0₄, or by treatment with strong reducing agents, such as sodium in liquid ammonia, sodium naphthalene,
- groups which form with the amine sulfonamides other than Tos-amides, e.g. including 2-nitrobenzenesulfonamide (nosyl) or o-nitrophenylsulfenyl (Nps), removable e.g.by treatment with samarium iodide, tributyltin hydride,
- benzylidene, removable e.g. by treatment with trifluoromethanesulfonic acid, trifluoroacetic acid, dimethyl sulfide;
- triphenylmethyl (trityl, Tr), dimethoxytrityl (DMT), e.g. removable by treatment with an acid, such as trifluoroacetic acid.

In a compound of formula IA or IB, respectively, preferably PROT is BOC and PROT' is hydrogen.

Alternatively PROT and PROT' together with the nitrogen atom to which they are attached form a heterocyclic ring, e.g. the nitrogen atom together with PROT and PROT' to which they are attached is part of a phthalimido ring; removable, e.g. by treatment with hydrazine.

In a further aspect in a compound of formula IA or IB a group PROT includes a group of formula -CO-R, wherein R together with CO to which it is attached is an amine protecting group, e.g. a electron withdrawing group, e.g. a leaving group, such as (C₁₋₈)alkoxy, e.g. tert.butoxy, or trihaloalkyl, e.g. CF₃ or C₆₋₁₂aryl, e.g. phenyl, e.g. including phenyl-carbonyloxy-methyl-phenyl, e.g. of formula 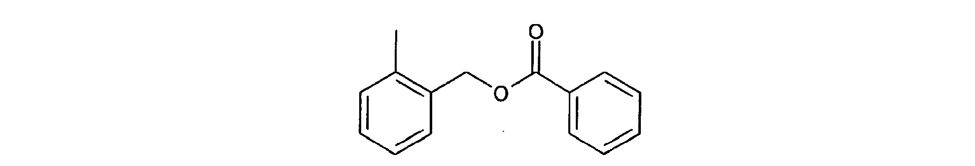 preferably in one aspect tert-butoxy, and/or CF₃; e.g. and PROT' preferably is hydrogen.

Appropriate amine protecting groups e.g. are described in T. W. Greene, P. G. M. Wuts, Protective Groups in Organic Synthesis, Wiley-Interscience, New York, 1999, particularly p. 503-607, 736-747.

PROT" is a thiol protecting group, e.g. including
- (C₁₋₆)alkyl, wherein alkyl optionally is further substituted, e.g. further substiuted by (C₆₋₁₂)aryl such as phenyl, such as trityl;
- (C₁₋₆)alkylcarbonyl, e.g. acetyl,
- (C₆₋₁₂)arylcarbonyl, such as a benzoyl, e.g. benzoyl;
   preferably (C₆₋₁₂)arylcarbonyl.

A compound of formula IA or IB, respectively, comprises a compound of formula 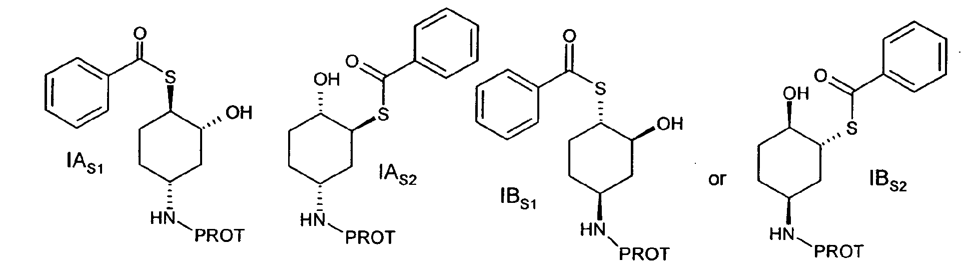 respectively, wherein PROT is as defined above; and in another aspect the present invention provides a compound of formula IA_{S1}, IA_{S2}, IB_{S}, or IB_{S1}, respectively, wherein PROT is an amine protecting group.

A compound of formula IA_{S1}, IA_{S2}, IB_{S1} or IB_{S2}, respectively, comprises a compound of formula 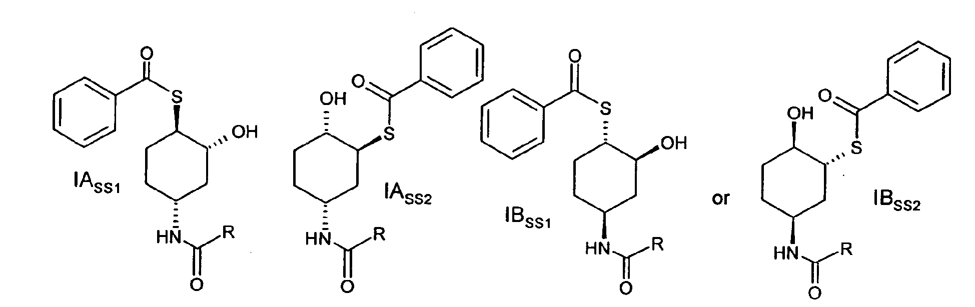 and in a further aspect the present invention provides a compound of formula IA_{SS1,} IA_{SS2}, IB_{SS1} or IB_{SS2}, respectively, wherein R is as defined above.

A compound of formula IA, or IB, respectively may be obtained by oxiran ring opening under introduction of a sulfur group in the side chain of the cyclohexyl ring in a compound of formula 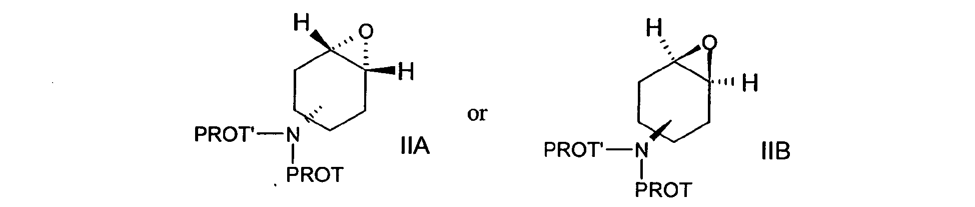 respectively, wherein PROT and PROT' are as defined above; e.g. by reaction with an optionally activated PROT"-thiol, wherein PROT" is defined as above.

Analogously as a compound of formula IA or IB, respectively, a compound of formula IA_{S1}, IA_{S2}, IB_{S1} or IB_{S2}, respectively, may be obtained by oxiran ring opening under introduction of a sulfur group in the side chain of the cyclohexyl ring in a compound of formula 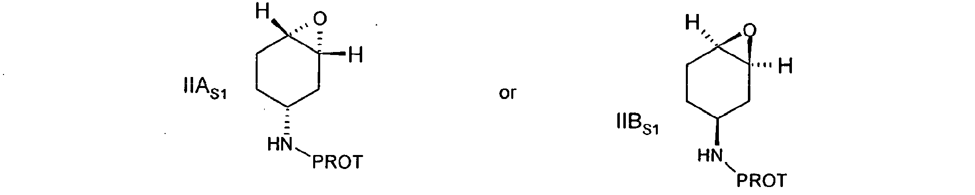 wherein PROT is as defined above.

Analogously as a compound of formula IA or IB, respectively, a compound of fomula IA_{SS1}, IA_{SS2}, IB_{SS1} or IB_{SS2}, respectively, may be obtained by oxiran ring opening under introduction of a sulfur group in the side chain of the cyclohexyl ring in a compound of formula 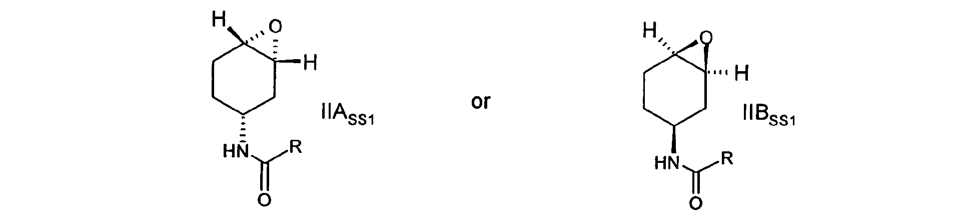 wherein R is as defined above.

Oxiran ring opening under introduction of a sulfur group in the side chain of the cyclohexyl ring in any of the compounds IIA, IIB, IIA_{S1}, IIB_{S1}, IIA_{SS1} or IIB _{SS1,} respectively, per se is a known reaction and may be performed as appropriate, e.g. according, e.g. analogously to a method as conventional, or as described herein. In one embodiment of the present invention ring opening is carried out by reacting a compound of formula IIA, IIB, IIA_{S1}, IIB _{S1}, IIA_{SS1} or IIB _{SS1} respectively, with a thiol, optionally in the presence of an activating agent, such as tetrabutylammonium chloride, in a solvent, e.g. organic solvent such as toluene or chlorobenzene.

A thiol which is useful in a process for oxiran ring opening according to the present invention includes e.g.
- a (C₁₋₆)alkyl-thiol, wherein alkyl optionally is further substituted, e.g. further substiuted by (C₆₋₁₂)aryl such as phenyl, such as a trityl-thiol;
- a (C₁₋₆)alkyl-thio acid, e.g. thioacetic acid,
- a (C₆₋₁₂)aryl-thio acid, such as a thiobenzoic acid, e.g. thiobenzoic acid;
preferably a thiobenzoic acid.

Regioisomers IA_{S1}, IA_{S2}, IB_{S1}, IB_{S2}, or IA_{SS1} 1A_{SS2} IB_{SS1}, IB_{SS2}, respectively may be obtained. Separation of the regioisomers is surprisingly easy and may be carried out by precipitation, e.g. by precipitating a solid and treating with a solvent mixture, e.g. a toluene/heptane mixture to obtain a compound IA_{S1} separated from IA_{S2}, or a compound 1B_{S1}, separated from IB_{S2}, respectively. The straightforwardness and simplicity of this separation is of particular relevance on an industrial scale and avoids cumbersome and expensive separation procedures e.g. separation via chromatography.

Compounds of formula IIA or IIB, or IIA_{S1} or IIB _{S1}, or IIA_{SS1} or IIB _{SS1} are novel and in a further aspect the present invention provides a compound of formula IIA, IIB, IIA_{S1}, IIB_{S1}, IIA_{SS1}, or IIB_{SS1}, wherein PROT, PROT' and R are as defined above.

A compound of formula IIA or IIB, or IIA_{S1} or IIB _{S1}, or IIA_{SS1} or IIB _{SS1}, respectively, may be obtained by epoxidation of the double bond in a compound of formula 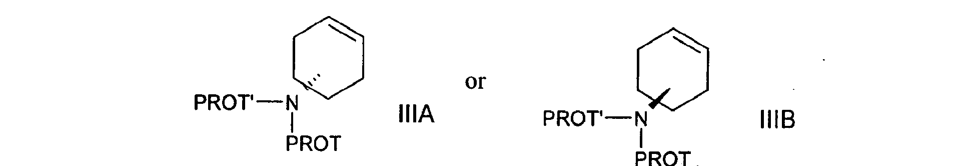 or of formula 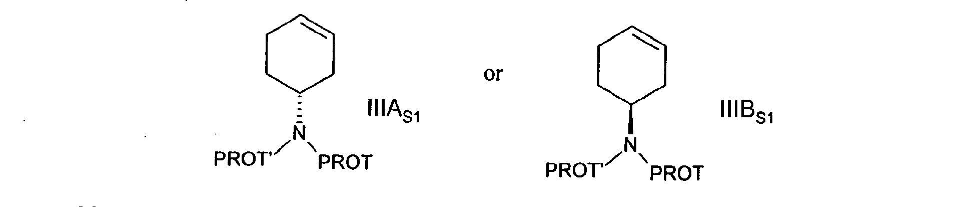 or of formula 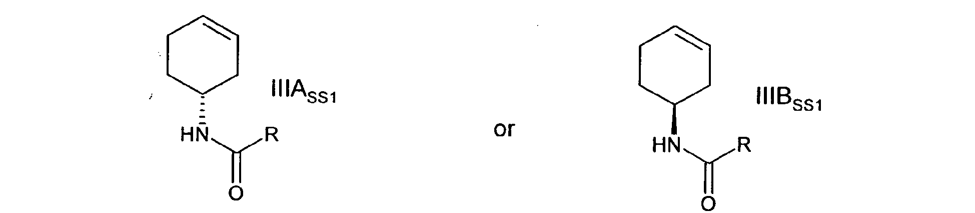 respectively, wherein PROT and PROT' and R are as defined above.

Epoxidation per se is a known process and may be carried out as appropriate, e.g. according, e.g. analogously to a method as conventional, or as described herein, e.g. by use of an oxidation agent, such as 3-chloroperbenzoic acid in a solvent, e.g. organic solvent, such as CH₂Cl₂, chlorobenzene or toluene.

It was found, that the epoxidation is in favour of the syn (cis) compounds - as expected and as described in literature (e.g. Gómez-Sánchez, E.; Marco-Contelles, J. Tetrahedron 2005, 61, 1207-1219). However, surprisingly the desired syn (cis) isomers can easily be isolated as a very pure compound after work up of the reaction mixture and isolation of the products. The content of the corresponding trans isomers in the desired product has been found to be below 5%, e.g. determined by e.g. NMR.

In another aspect the present invention provides a process for the production of a compound of formula IIA or IIB, or IIA_{S1} or IIB _{S1}, or IIA_{SS1} or IIB _{SS1}, respectively, wherein PROT, PROT' and R are as defined above, comprising epoxidizing the double bond in a compound of formula IIIA, IIIB, IIIA_{S1}, IIIB _{S1}, IIIA_{SS1} or IIIB _{SS1} respectively, wherein PROT and PROT' and R are as defined above, and isolating a compound of formula IIA or IIB, or IIA_{S1} or IIB _{S1}, or IIA_{SS1} or IIB _{SS1}, respectively, obtained from the reaction mixture.

Compounds of formula IIIA, IIIB, IIIA_{S1}, IIIB _{S1}, IIIA_{SS1} or IIIB _{SS1} with the exception of a compound of formula IIIB _{SS1}, wherein R is benzyloxycarbonyl, are novel and in a further aspect the present invention provides a compound of formula IIIA, IIIB, IIIA_{S1}, IIIB _{S1}, IIIA_{SS1} or IIIB _{SS1}, with the proviso that a compound of formula IIIB _{SS1}, wherein R is benzyloxycarbonyl is excluded.

Compounds of formula IIIA, IIIB, IIIA_{S1}, IIIB _{S1}, IIIA_{SS1} or IIIB _{SS1} may be obtained as appropriate, e.g. according, e.g. analogously, to a method as conventional or as described herein,
e.g. by protecting the amine group in a compound of formula 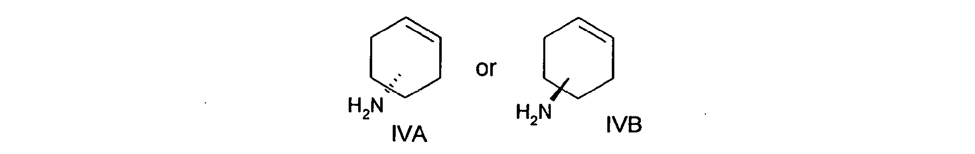 respectively, optionally in salt form, e.g. in the form of a hydrochloride, or in a compound of formula 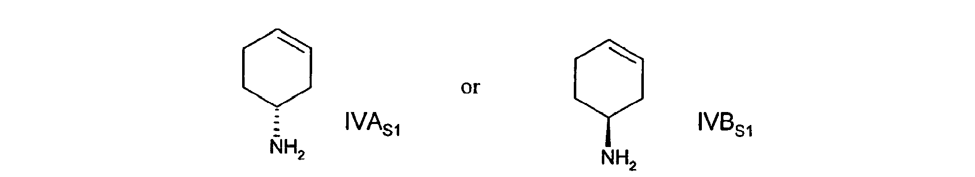 respectively, optionally in salt form, e.g. in the form of a hydrochloride,
- either with an amine protecting group PROT-L or PROT-PROT'-L, wherein L is an electron withdrawing group, e.g. a leaving group,
- or by acylation or carbamoylation,
   - e.g. by reaction with a an activated carboxylic acid or carboxylic acid derivative, such as an anhydride of a carboxylic acid, or carboxylic acid halogenide;
   - e.g. by reaction with an activated carboxylic acid or carboxylic acid derivative , wherein an electron withdrawing group is present, e.g. a leaving group, such as a compound of formula L-CO-R, wherein R and L are as defined above,
   - e.g. by reaction with an activated trihaloalkyl or C₆₋₁₂aryl carboxylic acid, or with an activated carboxylic acid (C₁₋₆)alkyl ester,
to obtain a compound of formula IIIA, IIIB, IIIA_{S1}, IIIB _{S1}, IIIA_{SS1} or IIIB _{SS1,} respectively.

A compound of formula IIIA_{SS1} or IIIB _{SS1},respectively wherein R is as defined above, may be obtained by carbamylation or by acylation of the amine group in a compound of formula IVA_{S1} or IVB_{S1}, respectively, optionally in salt form, such as in the form of a hydrochloride, respectively, e.g. by treatment of a compound of formula IVA_{S1} or IVB_{S1}, optionally in salt form, such as in the form of a hydrochloride, respectively, e.g. with an activated carboxylic acid or carboxylic acid derivative, such as an anhydride of the carboxylic acid or carboxylic acide halogenide, e.g. with an activated carboxylic acid or carboxylic acid derivative wherein an electron withdrawing group is present, e.g. with an activated trihaloalkyl or C₆₋₁₂aryl carboxylic acid, or with an activated carboxylic acid (C₁₋₆)alkyl ester.

Compounds of formula IVA, IVB, IVA_{S1} or IVB_{S1} are novel and in another aspect the present invention provides a compound of formula IVA, IVB, IVA_{S1} or IVB_{S1}, such as IVA, IVB or IVA_{S1}.

Compounds of formula IIIA_{S1} IIIVB _{S1}, IIIA_{SS1} or IIIB_{SS1} may also be obtained by a Curtius rearrangement from a compound of formula 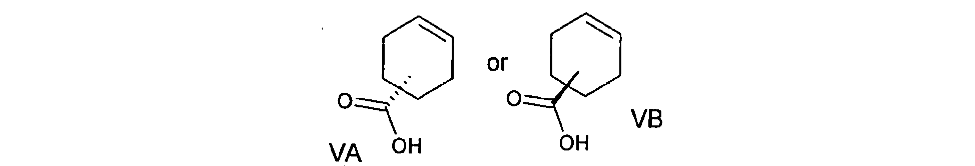 respectively, such as a compound of formula 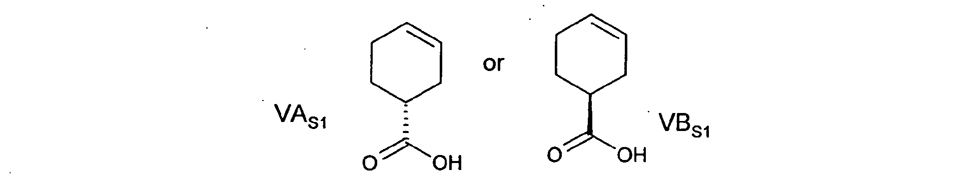 by treating a compound of formula VA, VB, VA_{S1} or VB_{S1}, respectively, with an azidoylation agent, namely an agent which is able to form an azidocarbonyl group from a carboxyl group, such as diphenylphosphoryl azide (DPPA), in the presence of a base, e.g. an amine, such as triethylamine, to form the azido carbonyl of formula 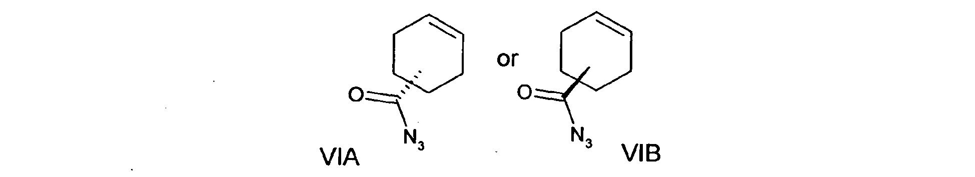 respectively, such as a compound of formula 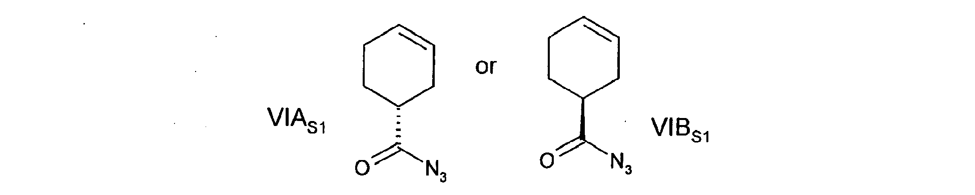 respectively, which under loss of nitrogen, rearranges to an isocyanate of formula 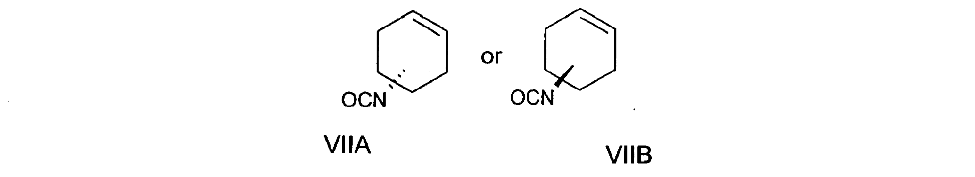 respectively, such as a compound of formula 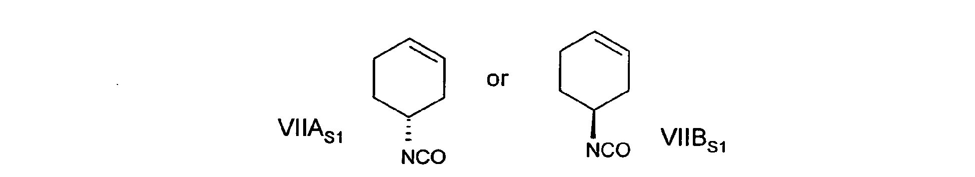 by treatment in organic solvent, such as CH₂C1₂, chlorobenzene or toluene, and treating the isocyanate obtained with an alcohol, such as a (C₁₋₈)alkylalcohol, such as a (C₁₋₆)alkylalcohol, optionally in the presence of CuCl (Kampferer, P.; Vasella, A. Helvetica Chimica Acta 2004, 87, 2764-2789), to obtain a compound of formula IIIA or IIIB or IIIA_{S1} or IIIB _{S1}. respectively, wherein PROT is -CO-C₁₋₈alkoxy, e.g. including -CO-C₁₋₆alkoxy, e.g. tert-butoxycarbonyl (BOC); or to obtain a compound of formula IIIA_{SS1} or IIIB _{SS1}, respectively, wherein R is C₁₋₈alkoxy, e.g. including C₁₋₆alkoxy, such as tert-butoxy.

In the Curtis rearrangement process provided according to the present invention compounds of formula VIA, VIB, VIA_{S1}, VIB_{S1}, or VIIA, VIIB, VIIA_{S1}, VIIB_{S1}, are not isolated.

Compounds of formula VA, VB, VA_{S1} or VB_{S1}, respectively may be obtained from a compound of formula 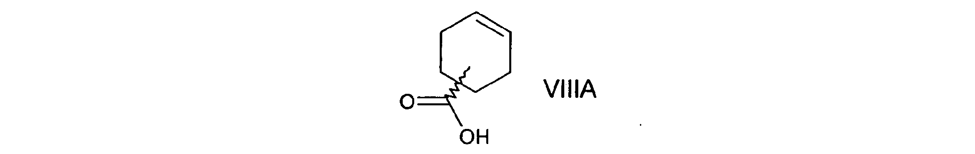 such as a compound of formula 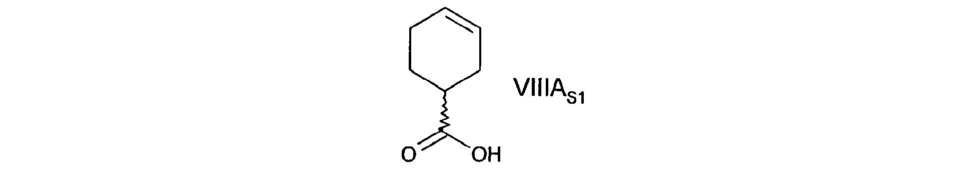 by enantiomeric separation e.g. via diastereomeric salts.

Separation of enantiomers of 3-cyclohexene carboxylic acid of formula VIIIA or VIIIA_{S1}, respectively, may be carried out by reaction with a chirally pure amine, e.g. reaction with (R)-(+)-methylbenylamine to obtain 3 -cyclohexene- S-carboxylic acid, or with (S)-(-)-methylbenylamine to obtain 3-cyclohexene-1R-carboxylic acid. The salt product obtained in such separation may be subjected to one or more recrystallizations until a required optical rotation (²⁰[α]_{D} > +40° for 3-cyclohexene-1R-carboxylic acid (S)-(-)-methylbenzylamine salt and ²⁰[α]_{D}> -40° for 3-cyclohexene-1S-carboxylic acid (R)-(+)-methylbenzylamine salt) is achieved.

The carboxylic acids of formula VIIIA or VIIIA_{S1,} respectively, may be provided by salt release under acidic conditions.

A compound of formula IIIA, IIIB, IIIA_{S1}, IIIB _{S1}, IIIA_{SS1}, or IIIB_{SS1} in a pure stereoisomeric form may also be obtained from a carboxylic acid of formula VA, VB, VA_{S1} or VB_{S1}, respectively, by reaction with an acid halogenide, e.g. acid chloride forming agent, e.g. (COCl)₂ to obtain the carboxylic acid chloride of formula 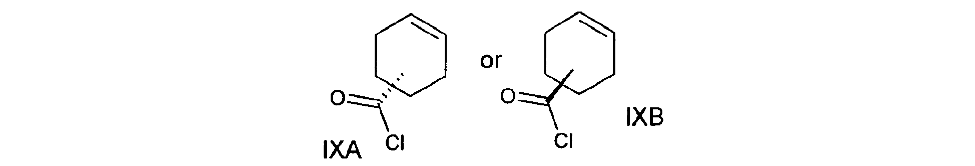 respectively, such as a compound of formula

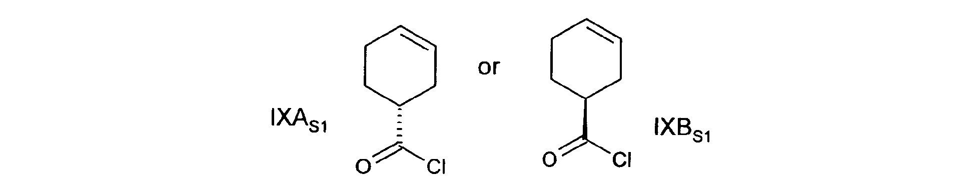

The acid chloride obtained may be further reacted twith NaN₃ to otain the corresponding acyl azide of formula VIA, VIB, VIA_{S1}, or VIB_{S1}, respectively.

The acyl azide of formula VIA, VIB, VIA_{S1}, or VIB_{S1}, respectively obtained may be subjected to a Curtius rearrangement to obtain the corresponding isocyanate of formula VIIA, VIIB, VIIA_{S1}, or VIIB_{S1}, respectively. The isocyanate of formula VIIA, VIIB, VIIA_{S1}, or VIIB_{S1}, respectively, obtained may be hydrolyzed, e.g. in the presence of an aqueous acid, e.g. HCl, to obtain a corresponding free amine optionally in the form of a salt, such as an acid addition salt, e.g. a hydrochloride of formula IVA, IVB, IVA_{S1}, or IVB_{S1}, respectively, or addition to the isocyanate may be carried out in the presence of an alcohol and optionally CuCl, to obtain a carboxylated amine of formula IIIA_{SS1} or IIIB_{SS1}, respectively; e.g. if tert-butyl alcohol is present as an alcohol the tert-butoxycarbonyl protected amine is obtained, e.g. if ethanol is present as an alcohol, the ethoxycarbonyl protected amine is obtained, in which amine or carboxylated amine is an amine or carboxylated amine according to the present invention of formula IIIAS1, or IIIBS1, wherein PROT is tert-butoxycarbonyl or ethoxycarbonyl; or of formula IIIA_{SS1} or IIIB_{SS1}, wherein R is tert-butoxy or ethyloxy.

Compounds of formula VIA, VIB, VIA_{S1}, VIB_{S1}, VIIA, VIIB, VIIA_{S1} VIIB_{S1}, IXA, IXB or IXB_{S1} are novel and in another aspect the present invention provides
- A compound of formula VIA, VIB, VIA_{S1}, or VIB_{S1}, respectively.
- A compound of formula VIIA, VIIB, VIIA_{S1}, or VIIB_{S1}, respectively.
- A compound of formula IXA, IXB or IXB_{S1}.

In another aspect the present invention provides a process for the production of a compound of formula IA, IB, IA_{S1}, IA_{S2}, IB_{S1}, IB_{S2}, IA_{SS1} IA_{SS2}, IB_{SS1} or IB_{SS2} respectively, wherein PROT, PROT' and R are as defined above, comprising
a) either reacting a compound of formula VA, VB, VA_{S1} or VB_{S1}, respectively, with an acid halogenide forming agent, such as (COC1)₂ to obtain the corresponding carboxylic acid chloride of formula IXA, IXB, IXA_{S1}, or IXB_{S1}, respectively, and reacting the carboxylic acid chloride obtained with NaN₃, or
   reacting a compound of formula VA, VB, VA_{S1} or VB_{S1}, respectively, with an azidoyl forming agent, e.g. diphenylphosphoryl azide, optionally in the presence of a base,
   to obtain the corresponding acyl azide of formula VIA, VIB, VIA_{S1}, or VIB_{S1,} respectively,
b) subjecting the acyl azide of step a) to a Curtius rearrangement to obtain a corresponding isocyanate of formula VIIA, VIIB, VIIA_{S1}, or VIIB_{S1}, respectively,
c) reacting the isocyanate of step b), optionally via hydrolysis and decarboxylation, in the presence of an aqueous acidic solution to obtain a compound of formula IVA, IVB, IVA_{S1} or IVB_{S1} which can optionally be protected to obtain a compound of formula IIIA_{S1}, IIIB _{S1} IIIA_{SS1} or IIIB _{SS1} or via addition of an alcohol and optionally in the presence of CuCl, to obtain a compound of formula IIIA_{S1} IIIB _{S1}, IIIA_{SS1} or IIIB _{SS1} respectively, wherein PROT and R are as defined above,
d) optionally replacing a group NH-PROT in a compound of formula IIIA_{S1}, IIIB _{S1}, IIIA_{SS1} or IIIB _{SS1} of step c) by a heterocyclic ring as an amine protecting group, which heterocyclic ring comprises PROT and PROT' and an amine group which amine group is attached to the cyclohexyl ring via its nitrogen atom, to obtain a compound of formula of formula IIIA or IIIB, respectively, wherein PROT and PROT' together with the nitrogen atom to which they are attached form a heterocyclic ring as an amine protecting group,
e) subjecting a compound of formula IIIA, IIIB, IIIA_{S1} IIIB _{S1}, IIIA_{SS1} or IIIB _{SS1}, of step c) or d) to epoxidation to obtain a compound of formula IIA, IIB, IIA_{S1}, IIB _{S1}, IIA_{SS1}or IIB _{SS1} respectively, wherein PROT, PROT' and R are as defined above,
f) opening the oxiran ring in a compound of formula IIA, IIB, IIA_{S1}, IIB _{S1}, IIA_{SS1}, or IIB_{SS1}, under introduction of a sulfur group in the side chain of the cyclohexyl ring to obtain a compound of formula IA, IB, IA_{S1}, IA_{S2}, IB_{S1}, IB_{S2}, IA_{SS1}, IA_{SS2}, IB_{SS1} or IB_{SS2}, respectively, wherein PROT, PROT' and R are as defined above, respectively,
g) isolating a compound of IA, IB, IA_{S1}, IA_{S2}, IB_{S1}, IB_{S2}, I_{ASS1}, IA_{SS2,} IB_{SS1}, or IB_{SS2}, respectively, wherein PROT, PROT' and R are as defined above obtained from the reaction mixture, optionally in the form of a salt, and,
h) optionally forming a salt of a compound obtained in f), or, if a compound in f) is obtained in the form of a salt, optionally forming the free base of said compound.

In another aspect the present invention provides a process for the production of a compound of formula IA or IB, IA_{S1}, IAS_{S2}, IB_{S1} or IB_{S2}, IA_{SS1}, IA_{SS2}, IB_{SS1} or IB_{SS2} respectively, wherein PROT' is hydrogen and PROT, and R are as defined above, comprising
a) reacting a compound of formula VA, VB, V A_{S1} or VB_{S1}, respectively with an azidoyl forming agent, e.g. diphenylphosphoryl azide, optionally in the presence of a base, to obtain the corresponding acyl azide of formula VIA, VIB, VIA_{S1} or VIB_{S1}, respectively,
b) subjecting the acyl azide of step a) to a Curtius rearrangement to obtain a corresponding isocyanate of formula VIIA, VIIB, VIIA_{S1} or VIIB_{S1}, respectively,
c) reacting the isocyanate of step b) via addition of an alcohol and optionally in the presence of CuCl to obtain a compound of formula IIIA_{S1}, IIIB _{S1}, IIIA_{SS1} or IIIB _{SS1}, respectively, wherein PROT and R are as defined above and PROT' is hydogen,
d) subjecting a compound of step c) without isolation to epoxidation to obtain a corresponding oxiran in solution of formula IIA or IIB, IIA_{S1}, IIA_{S2}, IIB_{S1} or IIB_{S2}, IIA_{SS1}, IIA_{SS2}, IIB_{SS1} or IIB_{SS2} respectively, wherein PROT' is hydrogen and PROT, and R are as defined above,
e) subjecting a compound of step d) without isolation to oxiran ring opening under introduction of a sulfur group in the side chain of the cyclohexyl ring and direct isolation of a compound of formula IA or IB, IA_{S1.} IA_{S2}, IB_{S1} or IB_{S2}, IA_{SS1}, IA_{SS2}, IB_{SS1} or IB_{SS2}, respectively, wherein PROT and R are as defined above and PROT' is hydrogen, by addition of an appropriate anti solvent;
optionally whereby the reaction a) to e) is performed in a single solvent (system), and/or whereby none of the intermediates obtained in a) to d) is isolated,
and isolating a compound of formula IA or IB, IA_{S1}, IA_{S2}, IB_{S1} or IB_{S2}, IA_{SS1}, IA_{SS2}, IB_{SS1} or IB_{SS2}, optionally in the form of a solid, from the reaction mixture.

In the above process preferably the reaction a) to e) is performed in a single solvent (system), such as toluene or chlorobenzene.

In the above process preferably none of the intermediates obtained in a) to d) is isolated, e.g. the product obtained in a) to d), respectively, is directly further reacted in solution and only a compound obtained in e), namely a compound of formula IA or IB, IA_{S1}, IA_{S2}, IB_{S1} or IB_{S2}, IA_{SS1,} IA_{SS2,} IB_{SS1} or IB_{SS2} is isolated, e.g in the form of a solid, from the reaction mixture.

The above processes starting from a compound of formula VA, VB, VA_{S1} or VB_{S1}, respectively, may provide a compound of IA or IB, IA_{S1}, IA_{S2}, IB_{S1} or IB_{S2}, IA_{SS1}, IA_{SS2}, IB_{SS1}, or IB_{SS2}, resepectively, starting from a compound of formula VA, VA_{S1} or VB_{S1}, respectively, without the necessity to use any chromatography and is therefore particularly useful in large scale manufacturing.

Compounds of formula
IA, IB, IA_{S1}, IA_{S2}, IB_{S1} or IB_{S2}, IA_{SS1}, IA_{SS2}, IB_{SS1} or IB_{SS2} (also designated herein as "compound(s) of formula I);
IIA, IIB, IIA_{S1}, IIA_{S2}, IIB_{S1} or IIB_{S2}, IIA_{SS1}, IIA_{SS2}, IIB_{SS1} or IIB_{SS2} (also designated herein as "compound(s) of formula II");
IIIA, IIIB, IIIA_{S1}, IIIB _{S1}, IIIA_{SS1} or IIIB _{SS1} (also designated herein as "compound(s) of formula III");
IVA, IVB, IV A_{S1}, or IVB_{S1} (also designated herein as "compound(s) of formula IV");
VA, VB, VA_{S1}, or VB_{S1} (also designated herein as "compound(s) of formula V");
VIA, VIB, VIA_{S1}, or VIB_{S1} (also designated herein as "compound(s) of formula VI");
VIIA, VIIB, VIIA_{S1}, or VIIB_{S1} (also designated herein as "compound(s) of formula VII"); or
IXA, IXB, IXA_{S1}, or IXB_{S1} (also designated herein as "compound(s) of formula IX");
are in stereoisomerically pure form as indicated in the formula drawings.

"Stereoisomerically pure form" as indicated in the drawings herein designates a form wherein the compound shows a diastereomeric or enantiomeric excess of ≥ 90% of the indicated stereochemistry.

Compounds of formula II, III, IV, V, VI, VII or IX, wherein the residues are as defined above, all are provided in a stereoisomerically pure form and are useful for the production of a compound of formula I in a stereoisomerically pure form.

In another aspect the present invention provides the use of a compound of formula II, III, IV, V, VI, VII or IX for the production of a compound of formula I.

Compounds of formula I are useful for the production of pharmaceutically active compounds as disclosed in W02008/113089.

A compound of formula I may be obtained from a compound of formula II, III, IV, V, VI, VII or IX, respectively as an intermediate without chromatography which constitutes an enormous advantage, e.g. particularly in a production process on technical scale.

In the following examples all temperatures are in °C and are uncorrected.

The following abbreviations are used:

| | |
|---|---|
| °C | degrees Celsius |
| ¹H NMR | proton nuclear magnetic resonance spectroscopy |
| ¹³C NMR | carbon nuclear magnetic resonance spectroscopy |
| [α]_{D} | specific optical rotation angle at 589nm |
| c | concentration in g/100 ml |
| DMF | N,N-dimethylformamide |
| DMSO | dimethylsulfoxide |
| DPPA | diphenyl phosphoryl azide |
| ESI+ | electrospray ionization in positive mode |
| ESI- | electrospray ionization in negative mode |
| Et | ethyl |
| EtOAc | ethyl acetate |
| h | hour(s) |
| HPLC | high performance liquid chromatography |
| min | minute(s) |
| mCPBA | 3-chloroperbenzoic acid |
| M | molarity |
| MeOH | methanol |
| mp | melting point |
| MS | mass spectrometry |
| MTBE | methyl tert-butyl ether |
| m/z | mass/charge ratio |
| rt | room temperature |
| TLC | thin layer chromatography |
| wt | weight |

### Example 1

### tert-Butyl cyclohex-3-enyl-1(R)-carbamate

### A. Salt Formation of cyclohex-3-ene-1-carboxylic acid

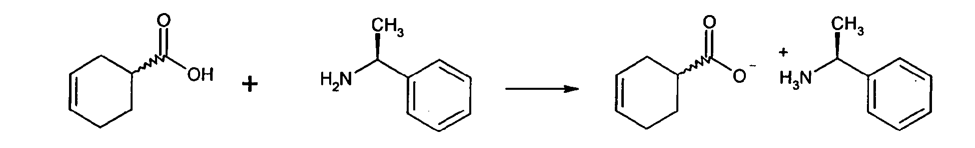

1000 g of racemic cyclohex-3-ene-1-carboxylic acid were charged to a flask and 5 volumes of acetone were added. The mixture obtained was stirred, heated to 55 to 60 °C and stirred for 30 min. To the mixture obtained 960.5 g of (S)-(-)-methylbenzylamine in 2 volumes of acetone were added dropwise over approximately 25 min. A clear, orange solution was obtained and cooled slowly. Crystallisation started at 53 °C (after 30 min). Full crystallisation occurred after ∼1h at 49 °C. The mixture obtained was cooled to rt over a further 3 h with an ice bath then stirred at rt for a further 1.5 h. The precipitate obtained was filtered off and washed with acetone. A methylbenzylamine salt of cyclohex-3-ene-1-carboxylic acid as set out in the reaction scheme above was obtained.

Yield (wet): 1966.9 g; optical rotation: ²⁰[α]_{D} = +8.05° (c=1, MeOH)

### B. Salt Resolution

1966.9 g (wet) of a salt as set out under step A and 3.8 volumes of acetone were charged to a 10 L vessel and heated to 55 to 60°C. When the product had dissolved, the reaction was stirred for a further 15 min and then slowly cooled to rt. Crystallisation started after 1 h 10 min (53°C). The mixture obtained was cooled to 20 to 25°C over 4.5 h and stirred at rt for a further 1.5 h. The precipitate obtained was filtered off and washed with acetone. A methylbenzylamine salt of cyclohex-3-ene-1-carboxylic acid wherein the R-isomer was enriched was obtained.

Yield (wet): 1143g; optical rotation: ²⁰[α]_{D} = +20.65° (c=1, MeOH)

Step B. was repeated until a required optical rotation (²⁰[α]_{D} > 40°) was achieved.

### C. Cyclohex-3-ene-1(R)-carboxylic acid

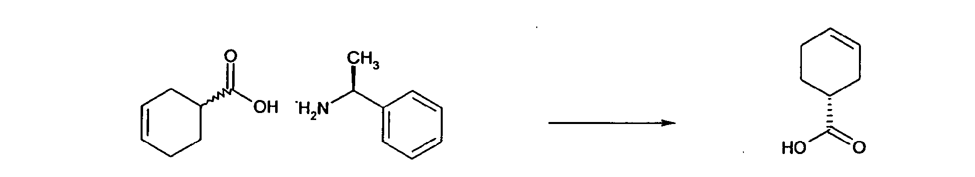

579.6 g of cyclohex-3-ene-1(*R*)-carboxylic acid (S)-(-)-methylbenzylamine salt and 5 volumes of MTBE were charged to a flask at 20-25°C and stirred. To the mixture obtained 10 volumes of 1M HCl were added, the mixture obtained was stirred for 5-10 min and two layers were formed. The layers obtained were separated and the aqueous layer was extracted with MTBE. The organic layers obtained were combined and washed with brine. The organic phase obtained was dried over Na₂SO₄, filtered, and the filter cake obtained was washed with MTBE. From the filtrate obtained solvent was removed in vacuo. Cyclohex-3-ene-1(*R*)-carboxylic acid in the form of a clear oil was obtained.
Yield: 301.78 g
Optical rotation ²⁰[α]_{D}= +83.1 ° (c=1, CHCl₃)

Cyclohex-3-ene-1(*R*)-carboxylic acid may be obtained in analogy to the method disclosed in Schwartz, H. M.; et al. JACS 1978, 100, 5199-5203.

### D. Curtius Rearrangement

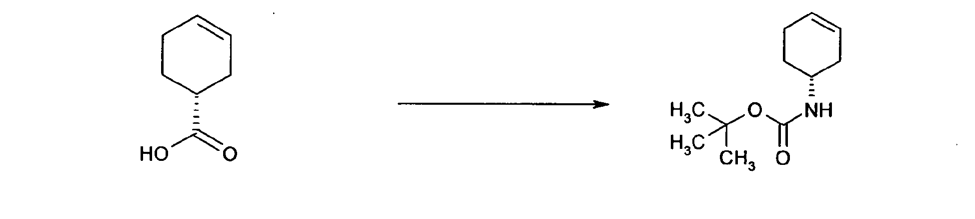

305 g of cyclohex-3-ene-1(*R*)-carboxylic acid and 10 volumes of toluene were charged to a flask at 20-25°C and stirred. To the mixture obtained 1.1 equivalents of NEt₃ were added dropwise over 15 min and the mixture obtained was stirred for a further 20 min. To the mixture obtained 1.0 equivalents of DPPA were added dropwise over approximately 20 minutes and the temperature raised to 95°C (exothermic reaction) with vigorous gas evolution. The mixture obtained was stirred for 15 min and heated to reflux. Progress of the reaction was followed by ¹HNMR measurements until completion. The mixture obtained was cooled to 80°C over 35 min and 5 equivalents of tert-butanol were added dropwise over 10 min, followed by 0.04 equivalents of CuCl. The mixture obtained was warmed to 100°C and stirred for a further 40 min. Progress of the reaction was followed by ¹H NMR measurements until completion. The mixture obtained was cooled and 5 volumes of aqueous, saturated NaHCO₃ solution were added over 10 min. The mixture obtained was stirred for 20 min and left overnight. The mixture obtained was filtered and the residual solid was washed with toluene. The organic layers were separated and the aqueous layer was washed with toluene. All organic layers obtained were combined, washed with H₂O and solvent was removed *in vacuo. tert-*Butyl cyclohex-3-enyl-1(*R*)-carbamate was obtained in the form of a light brown solid. Crude Yield: 479.7 g

The crude *tert*-Butyl cyclohex-3-enyl-1(R)-carbamate obtained was subjected to chromatography. For 160 g of crude product the column was packed with 1.5 Kg silica gel, using 2.5 L of cyclohexane, and topped with sand. The crude product was loaded in 0.8 L of 5% EtOAc/cyclohexane. The column was flashed with the following gradient system, a discrete fraction being collected each time:
2% EtOAc/cyclohexane (9 x 0.8L fractions)
5% EtOAc/ cyclohexane (7 x 0.8L fractions)
10% EtOAc/ cyclohexane (4 x 0.8L fractions)
Overall yield after chromatography: 81.3% of theory

¹H NMR (CDCl₃, 500 MHz, ppm): δ 5.64-5.67 (m, 1H), 5.56-5-60 (m, 1H), 4.54 (s, broad, 1H), 3.77 (s, broad, 1H), 2.32-2.34 (m, 1H), 2.07-2.17 (m, 2H), 1.81-1.87 (m, 2H), 1.48-1.56 (m, 1H), 1.44 (s, 9H)

¹³C NMR (CDCl₃, 500 MHz, ppm):): δ 155.3, 126.9, 124.5, 79.1, 45.7, 32.1, 28.4, 23.6

### Example 2

### tert-Butyl cyclohex-3-enyl-1(S)-carbamate

### A. Salt Formation

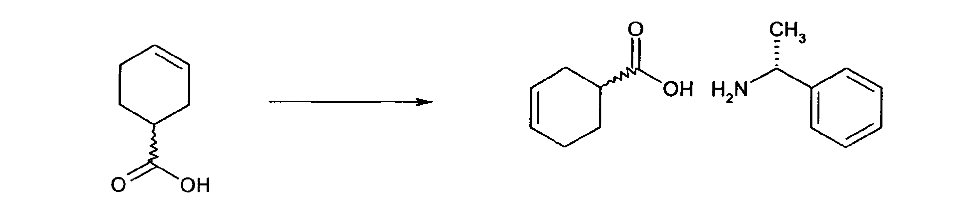

191.93 g of racemic cyclohex-3-ene-1-carboxylic acid were charged to a flask and 5 volumes of acetone were added. The mixture obtained was stirred, heated to 55 to 60°C and stirred for approximately 30 min. To the mixture obtained 1.0 equivalent of (*R*)-(+)-methylbenzylamine in 2 volumes of acetone were added dropwise over approximately 50 min. A clear, yellow solution was obtained and refluxed for 2 h. The solution obtained was cooled slowly to rt. Crystallisation started at approximately 50°C. Once at rt from the mixture obtained the precipitate was filtered off, washed with acetone and dried in vacuo at 40°C overnight. A methylbenzylamine salt of cyclohex-3-ene-l-carboxylic acid as set out in the reaction scheme above was obtained.
Yield: 251.76 g (66.9% of theory); Optical Rotation: [α]_{D} (c=5.12, MeOH) = -7.7°

### B. Resolution

A portion of the salt from step A and 5 volumes of acetone were charged to a flask and heated to reflux. Further acetone was added in order to ensure complete dissolution. When the product had dissolved, the mixture obtained was stirred for a further 15 min under reflux and slowly cooled to rt. The precipitate obtained was filtered off and washed with acetone. A methylbenzylamine salt of cyclohex-3-ene-l-carboxylic acid wherein the S-isomer was enriched was obtained.
Yield: Weight (wet): 161.15 g; optical rotation: ²⁰ [α]_{D} = -25.8° (c=5.16, MeOH)

The above procedure is repeated until required optical rotation (²⁰ [α]_{D} >-40°) is achieved.

### C. Salt Release

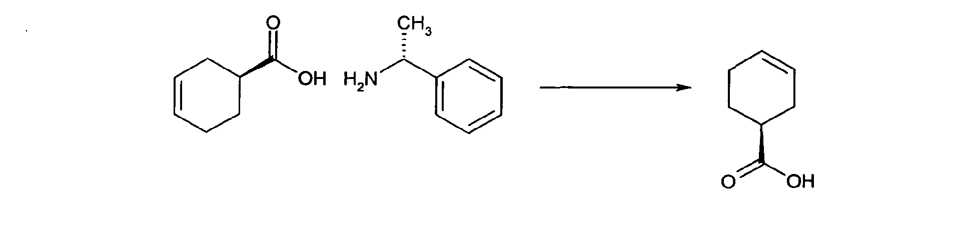

54.94 g of cyclohex-3-ene-1(*S*)-carboxylic acid (R)-(+)-methylbenzylamine salt and 18.2 volumes of H₂O were charged to a flask at 20-25°C and stirred. To the mixture obtained further 6.7 volumes of H₂O and 10 volumes of EtOAc were added. The mixture obtained was stirred for 15 min, the phases were separated and the organic layer was discarded. The aqueous layer was treated with 10 volumes of MTBE and 2.5 equivalents of 6M HCI were added slowly. The mixture obtained was stirred and two layers were formed, separated and the aqueous layer was extracted with MTBE. The organic layers obtained were combined and washed with aqueous, 30% NaCl solution. The organic phase obtained was dried over Na₂S0₄, filtered, and from the filtrate obtained solvent was removed in vacuo. Cyclohex-3-ene-1(*S*)-carboxylic acid in the form of a clear oil was obtained.
Yield: 26.13 g (93.3% of theory); Optical Rotation: [α]_{D} (c=4.66, MeOH) = -91.3°

Cyclohex-3-ene-1(*S*)-carboxylic acid may be obtained in analogy to the method disclosed in Schwartz, H. M.; et al. JACS 1978, 100, 5199-5203.

### 3. Curtius Rearrangement

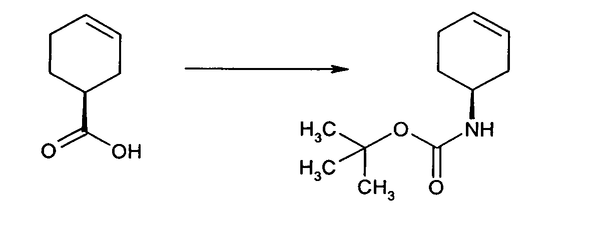

26.13 g of cyclohex-3-ene-1(*S*)-carboxylic acid and 14.9 volumes of toluene were charged to a flask at 20-25°C and stirred. To the mixture obtained 1.1equivalents of NEt₃ were added dropwise, followed by 1.0 equivalents of DPPA. The mixture obtained was heated to 60°C and stirred for 15 min and heated further to reflux. The progress of the reaction was followed by ¹H NMR measurments until completion. The mixture obtained was cooled to 50°C over 40 min and 5 equivalents of tert-butanol were added dropwise over 10 min, followed by 0.04 equivalents ofCuCl. The mixture obtained was heated to reflux. Progress of the reaction was followed by ¹H NMR measurments until completion. The mixture obtained was cooled and 5 volumes of aqueous, 30% NaHCO₃ solution were added. The mixture obtained was stirred for 15 min, filtered through celite in order to remove residual solid and the solid obtained was washed with toluene.The organic layers obtained were separated and the aqueous layer obtained was washed with toluene. All organic layers obtained were combined, washed with aqueous saturated NaCl solution, dried and solvent was removed in vacuo. *tert*-Butyl cyclohex-3-enyl-1(*S*)-carbamate was obtained in the form of a brown solid.
Crude Yield: 39.18 g; Optical Rotation: [α]_{D} (CHCl₃) = -16.7°

The crude product obtained was subjected to column chromatography (eluent: Cyclohexane/EtOAc 9:1). The required fractions were identified, combined and concentrated in vacuo. *tert-*Butyl cyclohex-3-enyl-1(*S*)-carbamate in the form of a white solid was obtained.
Yield: 62.2% of theory; Optical Rotation: [α]_{D} (c=5.70, CHC1₃) = -18.3°

¹H NMR (CDCl₃, 200 MHz, ppm): δ 5.52-5.68 (m, 2H), 4.56 (s, broad, 1H), 3.75 (s, broad, 1H), 2.29-2.42 (m, 1H), 2.07-2.11 (m, 2H), 1.76-1.90 (m, 2H), 1.48-1.56 (m, 1H), 1.43 (s, 9H)

### Example 3

### tert-Butyl cyclohex-3-enyl-1(R)-carbamate via Acid Chloride

### A. Cyclohex-3-ene-1(R)-carbonyl chloride

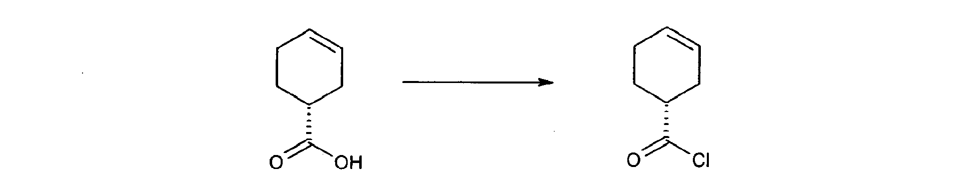

5 g of cyclohex-3-ene-1(*R*)-carboxylic acid were dissolved in diethyl ether and 1.2 equivalents of (COCl)₂ and two drops of DMF were added giving immediate gas evolution. After 1.5 h gas evolution had subsided and the solvent was removed in vacuo. Cyclohex-3-ene-1(*R*)-carbonyl chloride was obtained in the form of a light yellow oil.
Yield: 5.4 g (94.2% of theory); Optical Rotation: [α]_{D} (c=3.2, CHCl₃) = +79.8°.

### B. tert-Butyl cyclohex-3-envl-1 (R)-carbamate

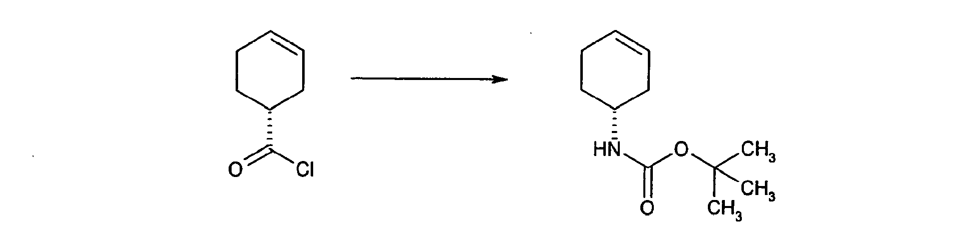

5.4 g of cyclohex-3-ene-1(*R*)-carbonyl chloride (see Doering, W,; et al. JACS 1972, 94, 3833-3844) in 9.3 volumes of CH₂Cl₂ were charged to a flask, and the mixture obtained was stirred under argon. To the mixture obtained 0.003 equivalents of tetrabutylammonium bromide were added and the mixture obtained was cooled to 0 to 5°C. To the mixture obtained 1.0 equivalents of NaN₃ in 1.5 volumes of H₂O were added dropwise over∼15 min at <10°C and the mixture obtained was stirred at 0 to 5°C for 2 h and allowed to warm to rt, the organic phase was separated and dried over MgSO₄, filtered, and the filtrate obtained was stirred under argon. To the mixture obtained 5 equivalents of tert-butanol and 0.03 equivalents of CuCl were added. The mixture obtained was stirred at rt overnight, washed twice with 0.1N HCl, twice with 5% aqueous NaHCO₃ solution and finally twice with H₂O. The organic phase obtained was concentrated, co-distilled with cyclohexane, the concentration/distillation residue obtained was stirred in EtOAc and the insoluble material was filtered off. The filtrate obtained was concentrated in vacuo. *tert*-Butyl cyclohex-3-enyl-1(*R*)-carbamate in the form of a light grey solid was obtained.
Yield: 5.49g; Optical Rotation: [α]_{D} (c=3.0, CHCl₃) = +21.2°.

The crude product was subjected to column chromatography (eluent: toluene) for analytical determination. The required fractions were identified, combined and concentrated in vacuo. *tert*-Butyl cyclohex-3-enyl-1(*R*)-carbamate in the form of a white solid was obtained. Optical Rotation: [α]_{D} =+18.7°.

The ¹H NMR pattern is identical with that of example 1 D.

### Example 4

### tert-Butyl (1S,3S,6R)-(7-oxabicyclo[4.1.0]hept-3-yl)-carbamate

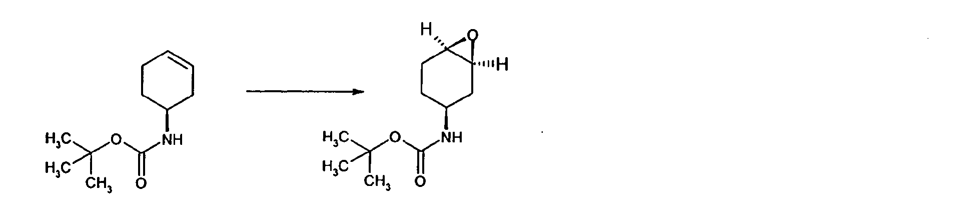

36.51 g of 70% mCPBA and 195 mL of CH₂Cl₂ were charged to a 1L flask and cooled using an ice bath. To the mixture obtained 24.34 g of *tert*-butyl cyclohex-3-enyl-1(*S*)-carbamate in 37 mL of CH₂Cl₂ were added dropwise over approximately 30 minutes ensuring a temperature below 30°C. 12 mL of CH₂Cl₂ were added and the mixture obtained was heated to reflux (40°C) until the reaction was complete (¹H NMR control). The mixture obtained was cooled to 0-5°C and the solid precipitate was filtered off and washed with CH₂Cl₂. The filtrate obtained was washed with 10% aqueous sodium thiosulfate in order to remove peroxides, saturated aqueous NaHCO₃ solution in order to ensure a pH>7 (recorded pH=9) in the aqueous phase, and water. The filtrate obtained was concentrated, 122 mL of toluene were added and the mixture obtained was concentrated again. A further portion of 122 mL of toluene was added and the mixture obtained was concentrated to approximately 2 volumes. The mixture obtained comprising *tert*-butyl (1*S*,3*S*,6*R*)-(7-oxabicyclo[4.1.0]hept-3-yl)-carbamate was kept cool (refrigerator) until required in the next step. From the solution_*tert-*butyl (1*S*,3*S*,6*R*)-(7-oxabicyclo[4.1.0]hept-3-yl)-carbamate₋was obtained in solid form upon concentration.
Yield: 17.27g

¹H NMR (200 MHz, DMSO-d₆, ppm) δ 6.63 (d, J=7Hz, 1H), 3.28 - 3.09 (m, 1H), 3.03 (s, broad, 2H), 2.15 - 1.96 (m, 2H), 1.85 - 1.67 (m, 2H), 1.59 - 1.27 (m, 11H)

### Example 5

### tert-Butyl (1R,3R,6S)-(7-oxabicyclo[4.1.0]hept-3-yl)-carbamate

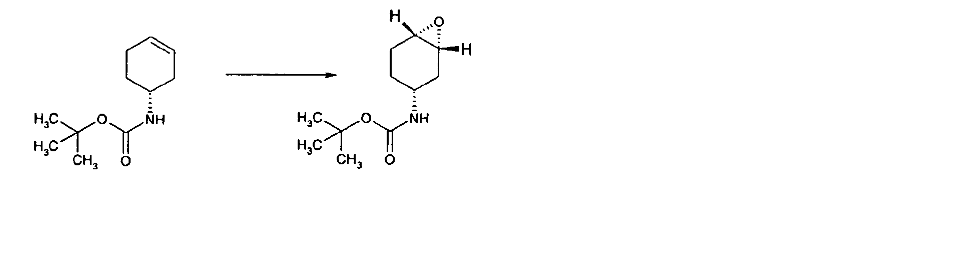

4500 g of mCPBA (70%) and 24 L of CH₂Cl₂ were charged to a vessel and cooled to 15°C. 3000 g of *tert*-butyl cyclohex-3-enyl-1(*R*)-carbamate in 4.5 L of of CH₂Cl₂ were added dropwise over approximately 30 min maintaining the temperature at 15 to 25°C. To the mixture obtained 1.5 L of CH₂Cl₂ were added and the mixture obtained was stirred at 20 to 25°C for 1 h and heated to reflux (40°C) for 2 h. Upon completion of the reaction (¹H NMR control), the mixture was cooled to -5 to 0°C, stirred for 30 min and the solid precipitate was filtered off and washed with CH₂Cl₂. The resultant filtrate was washed with 10% sodium thiosulfate solution in order to remove peroxides, saturated aqueous NHCO₃ solution until a pH>7 was achieved in the aqueous phase, and water. The organic phase obtained was concentrated to minimal volume and 15 L of toluene were added and the mixture was concentrated again to minimal volume. A further portion of 15 L of toluene was added and the mixture obtained was concentrated to approximately 2 volumes to produce *tert*-butyl (1*R*,3*R*,6*S*)-(7-oxabicyclo[4.1.0]hept-3-yl)-carbamate in the form of a solution in toluene. From the solution *tert*-butyl (1*R*,3*R*,6*S*)-(7-oxabicyclo[4.1.0]hept-3-yl)-carbamate was obtained in solid form upon concentration.
Crude Yield: 2.63 Kg
Pure Yield: 2.05 Kg

¹H NMR (200 MHz, CDCl₃, ppm) δ 4.85 (d, J=7Hz, 1H), 3.6 - 3.54 (m, 1H), 3.10 (s, broad, 2H), 2.23 - 1.99 (m, 2H), 1.92 - 1.67 (m, 2H), 1.54 - 1.27 (m, 11H)

### Example 6

### {(1S,2S,4S)-4-[(tert-Butoxycarbonyl)-amino]-2-hydroxy-cyclohexyl}-benzene-carbothioate and

### {(1R,2R,5S)-5-[(tert-Butoxycarbonyl)-amino]-2-hydroxy-cyclohexyl}-benzene-carbothioate

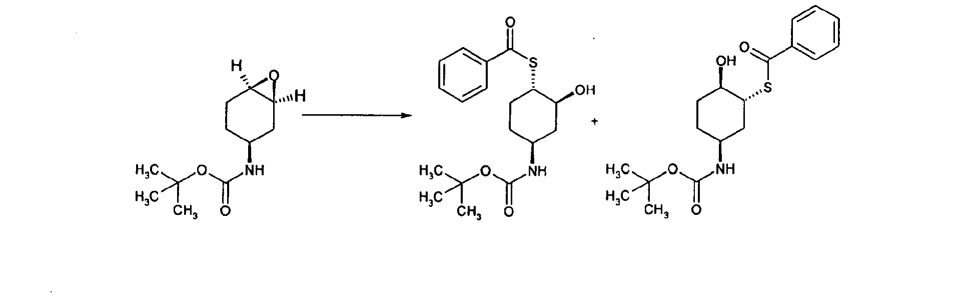

17.27 g of *tert*-butyl (1*S*,3*S*,6*R*)-(7-oxabicyclo[4.1.0]hept-3-yl)-carbamate and 52 mL of toluene were charged to a 1L flask and stirred at 15 to 25°C. To the mixture obtained 13 mL ofthiobenzoic acid (melted) were added dropwise. The temperature was kept below 30°C. Further 16 mL of toluene and 0.7g of tetrabutylammonium chloride monohydrate in one portion were added, external temperature control was stopped, and the mixture obtained was subjected to exotherm reaction. The mixture obtained was heated to 40-45°C. Upon completion of the reaction (TLC control), the mixture obtained was cooled to rt and washed with 5% aqueous NaHCO₃ solution followed by H₂O. The organic layer obtained was concentrated *in vacuo* to minimum volume. 86 mL of toluene were added and the mixture obtained was again concentrated to minimum volume. That process was repeated.
To the crude concentration residue obtained 17 mL of toluene were added under stirring, at which point a solid precipitated. 17 mL of heptane were added dropwise and the mixture was stirred at rt overnight. After stirring, the solid obtained was filtered off, dried and washed with 9 mL of toluene-heptane (1:1), followed by a slurry wash with 17 mL of toluene-heptane 1:1, followed by a further slurry wash with 35 mL of toluene-heptane 1:1. That procedure reduced the amount of regioisomer practically to undetectable amounts (determined by ¹H NMR). The regioisomer {(1*R*,2*R*,5*S*)-5-[(*tert*-butoxycarbonyl)-amino]-2-hydroxy-cyclohexyl}-benzene-carbothioate, which remains in solution can be isolated via conventional methods. The material which remained as crystalline solid was dried *in vacuo* at <40°C to give 9.41 g of {(1*S*,2*S*,4*S*)-4-[(*tert*-butoxycarbonyl)-amino]-2-hydroxycyclohexyl}-benzene-carbothioate in the form of a white solid.

Optical Rotation: [α]_{D} (c=3.73, CHCl₃) = +24.3°

{(1*S*,2*S*,4*S*)-4-[(*tert*-Butoxycarbonyl)-amino]-2-hydroxy-cyclohexyl}-benzene-carbothioate: ¹H NMR (200 MHz, DMSO-d₆, ppm) δ 7.92 - 7.87 (m, 2H), 7.71-7.63 (m, 1H), 7.58― 7.50 (m, 2H), 6.85 (d, J=8Hz, 1H), 5.11 (d, J=5.6Hz, 1H), 3.49- 3.25 (m, 3H), 2.12-1.95 (m, 2H), 1.79-1.69 (m, 1H), 1.54 - 1.14(m,12H)

### Example 7

### {(1R,2R,4R)-4-[(tert-Butoxycarbonyl)-amino]-2-hydroxy-cyclohexyl}-benzene-carbothioate and

### {(1S,2S,SR)-5-[(tert-Butoarycarbonyl)-amino]-2-hydroxy-cyclohexyl}-benzene-carbothioate

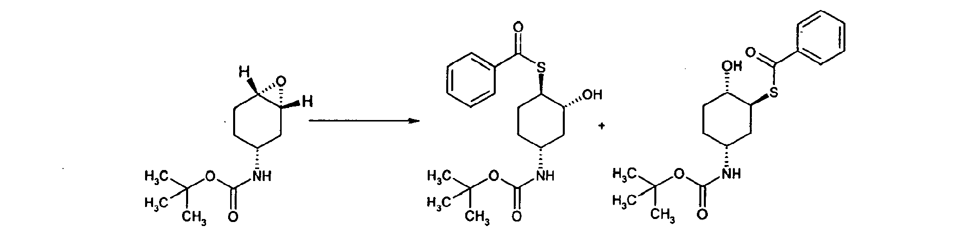

2050 g of *tert*-butyl (1*R*,3*R*,6*S*)-(7-oxabicyclo[4.1.0]hept-3-yl)-carbamate and 6.15 L of toluene were charged to a vessel and stirred at 15-25°C. To the mixture obtained 1.52 L of thiobenzoic acid (10%) were added dropwise. The temperature was kept below 30°C. Further 1.85 L of toluene and 85.5 g of tetrabutylammonium chloride monohydrate in one portion were added, external temperature control was stopped, and the mixture obtained was subjected to exotherm reaction. The mixture obtained was heated to 40-45°C and stirred for 4 h. Upon completion of the reaction (TLC and ¹H NMR-control), the mixture obtained was cooled to 15 to 20°C and washed with 5% aqueous NaHCO₃ solution followed by H₂O. The organic layer obtained was concentrated *in vacuo* to minimum volume. 10.25 L of toluene were added and the mixture obtained was again concentrated to minimum volume. That process was repeated.
To the crude concentration residue obtained 0.5 volumes of toluene were added under stirring and the mixture obtained was stirred at 15 to 25°C for 30 min. To the mixture obtained 0.5 volumes of heptane were added the mixture was stirred at 15 to 25°C for 1 h. After stirring, the solid obtained was filtered off, dried and washed with toluene-heptane (1:1), followed by a slurry wash toluene-heptane 1:1, followed by a further wash with toluene-heptane 1:1. That procedure reduced the amount of regioisomer practically to undetectable amounts (by ¹H NMR). The regioisomer {(1*S*,2*S*,5*R*)-5-[(*tert*-Butoxycarbonyl)-amino]-2-hydroxy-cyclohexyl}-benzene-carbothioate, which remains in solution can be isolated via conventional methods. The material which remained as solid was dried *in vacuo* at <40°C to give 3950 g of {(1*R*,2*R*,4*R*)-4-[(*tert*-Butoxycarbonyl)-amino]-2-hydroxycyclohexyl}-benzene-carbothioate in the form of a white solid.

### {(1R,2R,4R)-4-[(tert-Butoxycarbonyl)-amino]-2-hydroxy-cyclohexyl}-benzene-carbothioate:

¹H NMR (200 MHz, DMSO-d₆, ppm) δ 7.92 -7.87 (m, 2H), 7.71-7.63 (m, 1H), 7.58-7.,49 (m, 2H), 6.85 (d, J=8Hz, 1H), 5.11 (d, J=5.6Hz, 1H), 3.49― 3.25 (m, 3H), 2.12 -1.95 (m, 2H), 1.79-1.69 (m, 1H), 1.54-1.14 (m, 12H)

{(1 S,2S,5R)-5-[(tert-Butoxycarbonyl)-amino]-2-hydroxy-cyclohexyl}-benzene-carbothioate: ¹H NMR (200 MHz, DMSO-d₆, ppm) δ 7.92 (d, 2H), 7.75-7.68 (m, 1H), 7.61-7.53 (m, 2H), 6.92 (d, J=7.6Hz, 1H), 5.19 (d, J=3.2Hz, 1H), 3.93 - 3.91 (m, 1H), 3.66 (m, 1H), 3.48 - 3.38 (m, 1H), 2.18 - 2.07 (m, 1H), 1.80 - 1.39 (m, 5H), 1.39 (s, 9H)

### Example 8

### N-Cyclohex-3-enyl-2,2,2-trifluoro-acetamide

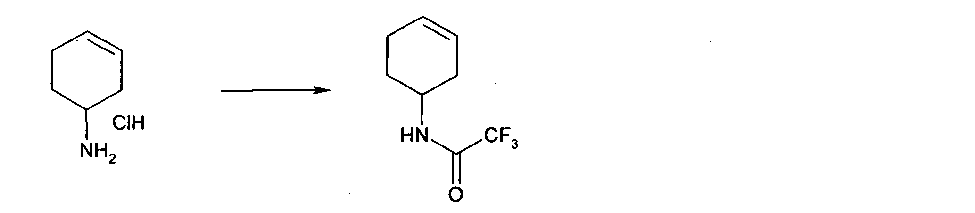

To a mixture of 19.4 g of the cyclohex-3-enylamine HCl-salt in the above scheme in 200 mL of CH₂Cl₂ and 50.5 mL of NEt₃ were added 28.3 mL of trifluoroacetic acid anhydride under argon at 0-5 °C under magnetic stirring over a period of 15 min. The mixture obtained was stirred for one h at 0-5 °C and for 5 h at rt, diluted with 200 mL of CH₂Cl₂ and washed twice with 1 M HCl (250 mL). The aqueous layers obtained were combined and extracted with 100 mL of CH₂Cl₂. The organic phases were combined, washed with saturated aq NaHCO₃ (250 mL); dried over MgSO₄ and concentrated in vacuo.

27.57 g of cyclohex-3-enyl-2,2,2-trifluoro-acetamide in the form of a white solid were obtained.

mp 55-58 °C

¹H-NMR (DMSO, 200 MHz, ppm): 9.33 (br d, 1H), 5.61 (m, 2H), 3.84 (m, 1H), 2.25-1.95 (m, 4H), 1.75 (m, 1H), 1.65-1.45 (m, 1H)

Following the procedure with chiral starting material gives chiral product.

### Example 9

### 2,2,2-Trifluoro-N-(7-oxa-bicyclo[4.1.0]hept-3-yl)-acetamide syn isomer

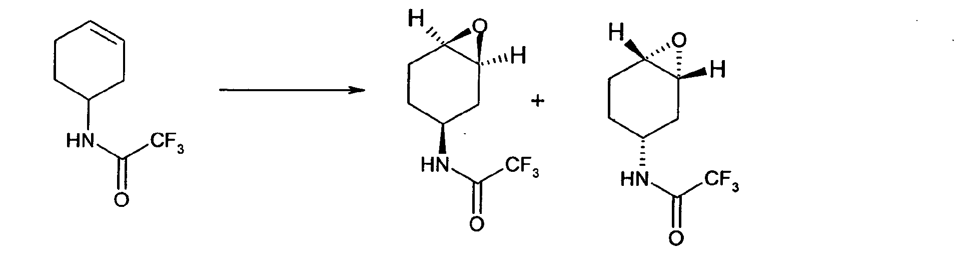

To a solution of 17 g of N-cyclohex-3-enyl-2,2,2-trifluoro-acetamide (Pfister, J., R.; Wymann, W., E. Synthesis 1983, 1, 38-40) in 170 mL of CH₂Cl₂, were added 22.8 g of *m*CPBA in one portion under magnetic stirring (exothermic). The mixture obtained was stirred for 2 hours under reflux (bath temperature 45 °C), the mixture obtained was cooled in an ice bath for 5 min, the precipitated solid obtained was filtered off and washed with CH₂Cl₂. The CH₂Cl₂ phase was washed twice with aqueous 1 M Na₂S₂O₃ (2 x 200 mL), three times with saturated aqueous NaHCO₃ (3 X 150 mL) and finally with 150 mL brine, dried over MgSO₄ and concentrated under vacuum.

17.66 g of 2,2-trifluoro-N-(7-oxa-bicyclo[4.1.0]hept-3-yl)-acetamide syn isomer in the form of a white solid were obtained.

¹H NMR (DMSO-d₆, 400 MHz, ppm): δ 9.16 (d, *J* = 7.04 Hz, 1 H), 3.65-3.54 (m, 1 H), 3.08 (br s, 2 H), 2.21-2.11 (m, 1 H), 2.09-2.00 (m, 1 H), 1.89-1.78 (m, 1 H), 1.71 (dd, *J* = 4, 11.02 Hz, 1 H), 1.45-1.33 (m, 2 H).

MS: ESI+ = 210.0 (M⁺), ESI- = 207.98 (M⁻).

mp of crude product: 77-85 °C

Following the procedure with chiral starting material gives chiral product.

### Example 10

### Chiral Acid to Thiobenzoate (Telescoped Procedure)

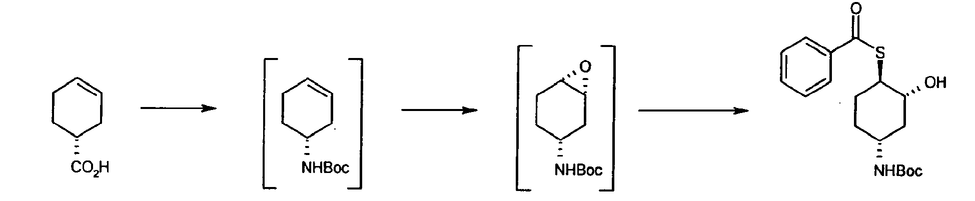

### A. Curtius Rearrangement to obtain tert-butyl cyclohex-3-enyl-1(R)-carbamate

To a solution of 150 g of cyclohex-3-ene-1*R*-carboxylic acid in 1275 mL of chlorobenzene were added 334.8 mL of NEt₃ and rinsed through with 75 mL of chlorobenzene. The mixture obtained was heated to 78 to 82°C and 327.2 g of DPPA were added cautiously, maintaining the temperature at 80 to 90 C, followed by 60 mL of chlorobenzene. The mixture obtained was stirred at 78 to 82°C for 1 h and analysed for completion by TLC (absence of starting material).
Upon completion 441 g of tert-butanol in 102 mL of chlorobenzene were charged to the mixture, maintaining the temperature at 70 to 80°C. 4.71 g of CuCl in 48 mL of chlorobenzene were added to the mixture obtained, followed by 27 mL of chlorobenzene as a line rinse. The mixture obtained was stirred at 90 to 95 °C for 2 h and analysed for completion by TLC (formation of product).
Upon completion, the reaction mixture was cooled to 15 to 25 °C, washed with 1500 mL of 20% aqueous K₂CO₃ solution, filtered through celite and rinsed with 150 mL of chlorobenzene to give a three phase mixture. The upper layer was washed again with 1500 mL of 20% aqueous K₂CO₃ solution, again giving three layers. The combined middle phases were extracted with 300 mL of chlorobenzene, the upper layer then being combined with the previous upper layer and washed with 1500 mL of 25% aqueous NaCl solution. The organic layer obtained was washed with 0.5 M H₃PO₄ (2 x 1500 mL) followed by 1500 mL of 5% aqueous NaCl solution. The content of the step A product in solution is determined via NMR or HPLC.

### B. Epoxidation to obtain tert-butyl (1R,3R,6S)-(7-oxabicyclo[4.1.0]hept-3-yl)-carbamate

The Curtius solution of step A in chlorobenzene (189.3 g olefinic material; assuming all Curtius), and 189 mL of chlorobenzene were cooled to 10 to 15°C and 284.0 g of 70% mCPBA were added portionwise, maintaining the reaction temperature below 30 °C. The mixture obtained was stirred at 20 to 25 °C for 1 h and analysed for completion by NMR (absence of starting material).
Upon completion the reaction mixture was heated to 38 to 42 °C, stirred for 2 h and analysed for completion by NMR (absence of trans-epoxide).
Upon completion, the reaction mixture was cooled to -5 to 0 °C, filtered and rinsed through with chlorobenzene (2 x 95 mL), warmed to 15 to 25 °C and washed with 10 % aqueous sodium thiosulfate solution (2 x 947 mL) followed by 5 % aqueous NaHCO₃ solution (3 x 947 mL) and finally H₂O (2 x 947 mL). The content of the step B product in solution is determined via NMR or HPLC.

### C. Ring-Opening to obtain {(1R,2R,4R)-4-[(tert-butoxycarbonyl)-amino]-2-hydroxycyclohexyl}-benzene-carbothioate

An epoxide solution in chlorobenzene from step B (124.9 g epoxide and of 25 mL of chlorobenzene) was de-gassed with argon for 30 min and the mixture obtained was adjusted to 15 to 20 °C. To the mixture obtained 88.0 mL of 90 % thiobenzoic acid were charged dropwise, maintaining the temperature below 30 °C, followed by 37 mL of chlorobenzene and 5.2 g of tetrabutylammonium chloride monohydrate, maintaining the temperature below 30 °C. The mixture obtained was stirred at 40 to 45 °C for 4 h and analysed for completion by NMR (absence of epoxide starting material).
Upon completion the reaction mixture was cooled to 15 to 20 °C and washed with 5 % aqueous NaHCO₃ solution (2 x 625 mL) follwed by H₂O (2 x 625 mL).
The batch obtained was concentrated at less than 45 °C to approximately 6.8 volumes and stirred at 20 to 25 °C until crystallisation initiated. To the mixture obtained 333 mL of heptane were added dropwise and the mixture obtained was stirred for a further 30 min. To the mixture obtained again 333 mL of heptane were added dropwise followed by a further 30 min stirring at 20 to 25 °C, and a final heptane charge of 333 mL is added. The mixture obtained was stirred at 20 to 25 °C for 16 h. The mixture obtained was filtered, the resultant cake washed with toluene-heptane (1:1, 4 x 124 mL) and dried *in vacuo* at <40 °C to give an off-white solid.
{( 1*R*,2*R*,4*R*)-4-[(*tert*-Butoxycarbonyl)-amino]-2-hydroxy-cyclohexyl }-benzene-carbothioate was obtained. Overall yield (from cyclohex-3-ene-1(*R*)-carboxylic acid): 75.2 g

## Claims

1. A compound of formula 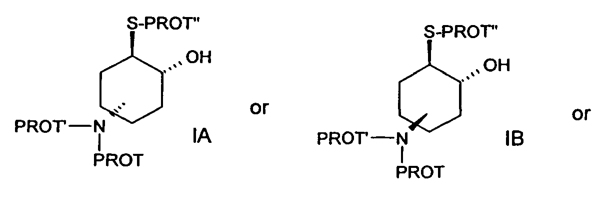 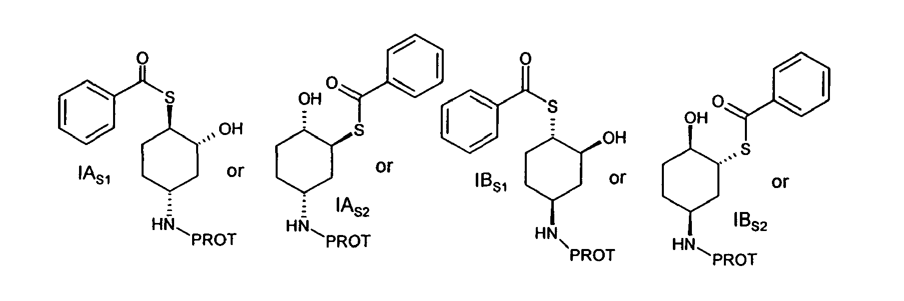 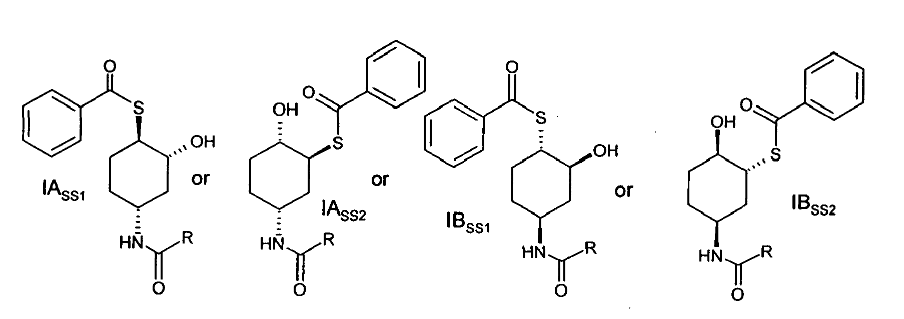 wherein
PROT is an amine protecting group and PROT' is hydrogen; or
PROT and PROT' together with the nitrogen atom to which are attached form a heterocyclic ring as an amine protecting group,
PROT" is a thiol protecting group, and
R together with the C=O to which it is attached is an amine protecting group; optionally R is an electron withdrawing group.

2. A process for the preparation of a copmpound of formula IA, IB, IA_{S1}, IB_{S1}, IA_{SS1}, or IB_{SS1}, respectively, as defined in claim 1, comprising oxiran ring opening in a compound of formula 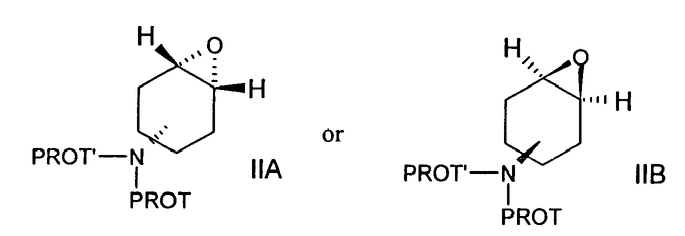 or in a compound of formula 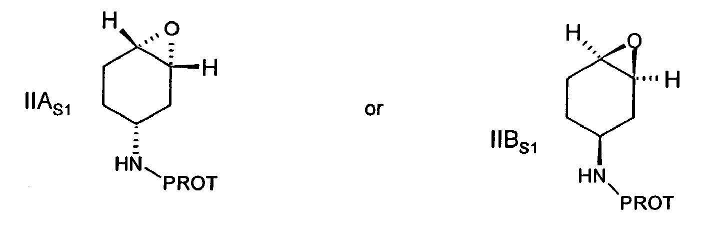 or in a compound of formula 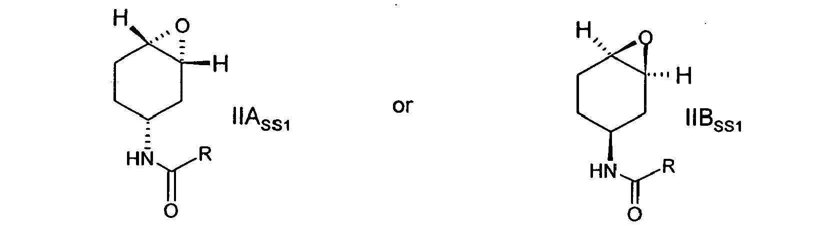 respectively, wherein PROT, PROT' and R are as defined in claim 1,
optionally by reaction with an optionally protected or activated thiol;
wherein optionally a compound of formula IIA, IIB, IIA_{S1}, IIB_{S1}, IIA_{SS1}, or IIB_{SS1} wherein PROT, PROT' and R are as defined in claim 1 is obtained by epoxidation of the double bond in a compound of formula IIIA, IIIB, IIIA_{S1}, IIIB _{S1}, IIIA_{SS1} or IIIB_{SS1}, respectively, as defined in claim 4, wherein PROT and PROT' and R are as defined above, and isolating a compound of formula IIA or IIB, or IIA_{S1} or IIB _{S1}, or IIA_{SS1}, or IIB _{SS1}, respectively, wherein PROT, PROT' and R are as defined above, obtained from the reaction mixture.

3. A compound of formula IIA, IIB, IIA_{S1}, IIB_{S1}, IIA_{SS1}, or IIB_{SS1}, according to claim 2, wherein PROT, PROT' and R are as defined in claims 1 and 2.

4. A compound of formula 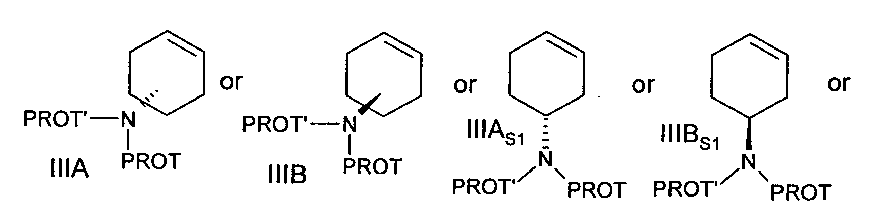 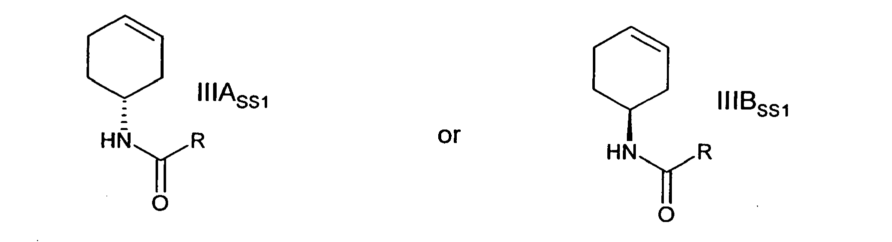 wherein PROT and PROT' and R are as defined in claims 1 and 3; with the proviso that a compound of formula IIIB_{SS1}, wherein R is benzyloxycarbonyl is excluded.

5. A compound of formula 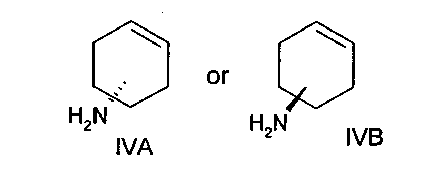 or of formula 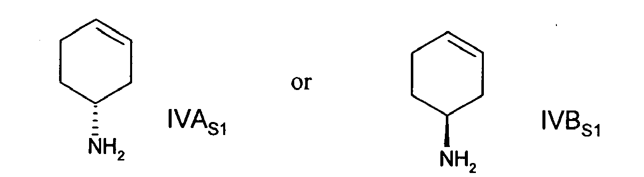 optionally in salt form, optionally in the form of a hydrochloride.

6. A compound of formula

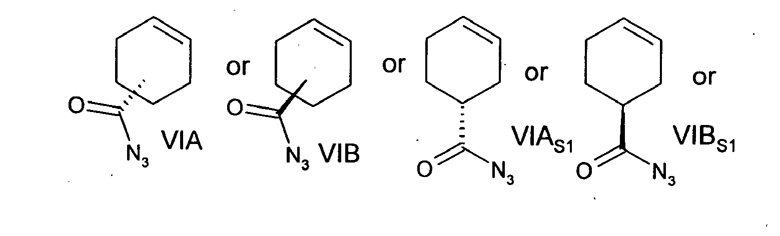

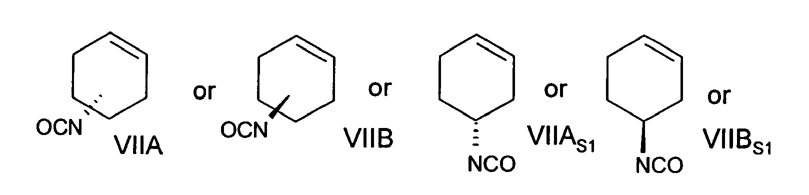

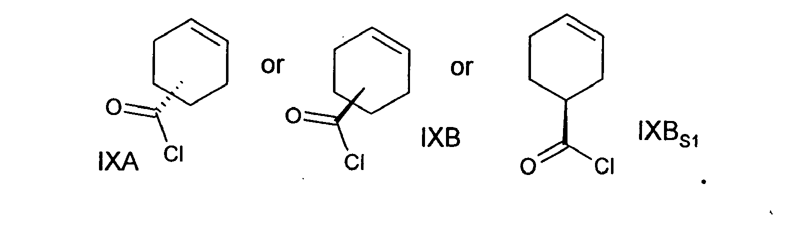

7. A process for the production of a compound of formula IA, IB, IA_{S1}, IA_{S2}, IB_{S1}, IB_{S2}, IA_{SS1}, IA_{SS2}, IB_{SS1} or IB_{SS2}, respectively, according to claim 1, wherein PROT, PROT' and R are as defined in claim 1, comprising
a) either reacting a compound of formula VA, VB, VA_{S1} or VB_{S1} respectively, according to claim 6, with an acid chloride forming agent, to obtain the corresponding carboxylic acid chloride of formula IXA, IXB, IXA_{S1}, or IXB_{S1}, respectively, according to claim 6, and reacting the carboxylic acid chloride obtained with NaN₃, or
reacting a compound of formula VA, VB, VA_{S1} or VB_{S1}, as defined in claim 6, respectively, with an azidoyl forming agent, which is optionally diphenylphosphoryl azide, optionally in the presence of a base,
to obtain the corresponding acyl azide of formula VIA, VIB, VIA_{S1} or VIB_{S1}, respectively, according to claim 6,
b) subjecting the acyl azide of step a) to a Curtius rearrangement to obtain a corresponding isocyanate of formula VIIA, VIIB, VIIA_{S1}, or VIIB_{S1}, respectively, according to claim 6,
c) reacting the isocyanate of step b), optionally via hydrolysis and decarboxylation, in the presence of an aqueous acidic solution to obtain a compound of formula IVA, IVB, IVA_{S1} or IVB_{S1}, which can optionally be protected to obtain a compound of formula IIIA_{S1}, IIIB_{S1}, IIIA_{SS1} or IIIB_{SS1}, or via addition of an alcohol and optionally in the presence of CuCl, to obtain a compound of formula IIIA_{S1}, IIIB _{S1}, IIIA_{SS1} or IIIB_{SS1}, respectively, according to claim 4, wherein PROT, PROT' and R are as defined in claim 4,
d) optionally replacing a group ―NH-PROT in a compound of formula IIIA_{S1}, IIIB_{S1}, IIIA_{SS1} or IIIB_{SS1}, according to claim 4, wherein PROT, PROT' and R are as defined in claim 4, of step c) by a heterocyclic ring as an amine protecting group, which heterocyclic ring comprises PROT and PROT' and an amine group which amine group is attached to the cyclohexyl ring via its nitrogen atom, to obtain a compound of formula of formula IIIA or IIIB, respectively, according to claim 4, wherein PROT and PROT' are as defined in claim 1
e) subjecting a compound of formula IIIA, IIIB, IIIA_{S1}, IIIB _{S1}, IIIA_{SS1}, or IIIB _{SS1} of step c) to epoxidation to obtain a compound of formula IIA, IIB, IIA_{S1}, IIB _{S1}, IIA_{SS1} or IIB _{SS1} respectively, according to claim 1, wherein PROT, PROT' and R are as defined in claim 1,
f) opening the oxiran ring in a compound of formula IIA, IIB, IIA_{S1}, IIB _{S1}, IIA_{SS1} or IIB_{SS1} as defined in claim 2, under introduction of a sulfur group in the side chain of the cyclohexyl ring to obtain a compound of formula IA, IB, IA_{S1}, IA_{S2}, IB_{S1}, IB_{S2}, IA_{SS1}, IA_{SS2}, IB_{SS1} or IB_{SS2}, respectively, according to claim 1, wherein PROT, PROT' and R are as defined in claim 1, respectively,
g) isolating a compound of formula IA, IB, IA_{S1}, IA_{S2}, IB_{S1}, IB_{S2,} IA_{SS1}, IA_{SS2}, IB_{SS1} or IB_{SS2}, respectively, wherein PROT, PROT' and R are as defined in claim 1, obtained from the reaction mixture, optionally in the form of a salt, and,
h) optionally forming a salt of a compound obtained in f), or, if a compound in f) is obtained in the form of a salt, optionally forming the free base of said compound.

8. A process for the production of a compound of formula IA or IB, IA_{S1}, IA_{S2}, IB_{S1} or IB_{S2,} IA_{SS1}, IA_{SS2}, IB_{SS1} or IB_{SS2} respectively, according to claim 1, wherein PROT and R are as defined in claim 1, and PROT' is hydrogen, comprising
a) reacting a compound of formula VA, VB, VA_{S1} or VB_{S1}, respectively, according to claim 6, with an azidoyl forming agent, e.g. diphenylphosphoryl azide, optionally in the presence of a base,
to obtain the corresponding acyl azide of formula VIA, VIB, VIA_{S1}, or VIB_{S1}, respectively, according to claim 6,
b) subjecting the acyl azide of step a) to a Curtius rearrangement to obtain a corresponding isocyanate of formula VIIA, VIIB, VIIA_{S1}, or VIIB_{S1}, respectively, according to claim 6,
c) reacting the isocyanate of step b) via addition of an alcohol and optionally in the presence of CuCl to obtain a compound of formula IIIA_{S1}, IIIB _{S1}, IIIA_{SS1}, or IIIB _{SS1}, respectively, according to claim 4, wherein PROT, and R are as defined in claim 1, and PROT' is hydrogen,
d) subjecting a compound of step c) without isolation to epoxidation to obtain a corresponding oxiran in solution of formula IIA or IIB, IIA_{S1}, IIA_{S2}, IIB_{S1} or IIB_{S2}, IIA_{SS1}, IIA_{SS2}, IIB_{SS1}, or IIB_{SS2} respectively, according to claim 2, wherein PROT and R are as defined in claim 1, and PROT' is hydrogen,
e) subjecting a compound of step d) without isolation to oxiran ring opening under introduction of a sulfur group in the side chain of the cyclohexyl ring, and
f) direct isolating a compound of formula IA or IB, IA_{S1}, IA_{S2}, IB_{S1} or IB_{S2}, IA_{SS1}, IA_{SS2}, IB_{SS1} or IB_{SS2}, respectively, according to claim 1, wherein PROT and R are as defined in claim 1 and PROT' is hydrogen,
by addition of an appropriate anti solvent,
optionally whereby the reaction a) to e) is performed in a single solvent (system), and/or whereby none of the intermediates obtained in a) to d) is isolated,
and isolating a compound of formula IA or IB, IA_{S1}, IA_{S2}, IB_{S1} or IB_{S2}, IA_{SS1}, IA_{SS2}, IB_{SS1} or IB_{SS2}, optionally in the form of a solid, from the reaction mixture.

9. Use of a compound of formula II, III, IV, V, VI, VII and/or IX according to any of claims 2, 4, 5 or 6, wherein PROT, PROT' and R are as defined in claim 1, for the production of a compound of formula I according to claim 1, wherein PROT, PROT' and R are as defined in claim 1.

10. A process for the production of a compound of formula IIA or IIB, or IIA_{S1} or IIB _{S1}, or IIA_{SS1} or IIB _{SS1}, respectively, as defined in claim 2, wherein PROT, PROT' and R are as defined in claim 1, comprising epoxidizing the double bond in a compound of formula IIIA, IIIB, IIIA_{S1}, IIIB _{S1}, IIIA_{SS1} or IIIB _{SS1}, respectively, as defined in claim 4, wherein PROT and PROT' and R are as defined above, and isolating a compound of formula IIA or IIB, or IIA_{S1} or IIB _{S1}, or IIA_{SS1} or IIB _{SS1}, respectively, wherein PROT, PROT' and R are as defined above, obtained from the reaction mixture.
